# EUROPEAN PATENT SPECIFICATION

(11) **EP 2 579 871 B1**
(45) Date of publication and mention of the grant of the patent: **26.08.2015**
(21) Application number: 11726022.4
(22) Date of filing: 07.06.2011
(51) Int. Cl.: A61K 31/375, A61K 31/675, A61P 31/22

(54) **TOPICAL ANTIVIRAL FORMULATIONS FOR PREVENTION OF TRANSMISSION OF HSV-2**
TOPISCHE ANTIVIRALE ZUBEREITUNGEN ZUR PRÄVENTION VON HSV-2 ÜBERTRAGUNG
PRÉPARATIONS ANTIVIRALES TOPIQUES POUR INHIBIBER LA TRANSMISSION DU HSV-2

(30) Priority: 22.12.2010 US 201061426373 P; 23.06.2010 US 357892 P; 11.06.2010 US 354050 P
(43) Date of publication of application: 17.04.2013
(73) Proprietor: Gilead Sciences, Inc., Foster City, CA 94404 (US); Abdool Karim, Quarraisha, 4013 Durban (ZA); Abdool Karim, Salim S., 4013 Durban (ZA); Kharsany, Ayesha, 4013 Durban (ZA)
(72) Inventor: ABDOOL KARIM, Quarraisha, 4013 Durban (ZA); ABDOOL KARIM, Salim, S., 4013 Durban (ZA); KHARSANY, Ayesha, 4013 Durban (ZA); CIHLAR, Tomas, Foster City CA 94404 (US); ROONEY, James, Francis, Portola Valley CA 94028 (US)
(74) Representative: Hallybone, Huw George
(86) International application number: PCT/US2011/039505
(87) International publication number: WO 2011/156416

(56) References cited:
- WO-A1-2009/127825
- WO-A2-03/082193
- WO-A2-2006/017044
- WO-A2-2008/148018
- WO-A2-2009/009625
- FHI and the Centre for the AIDS Programme of Research in South Africa: "Factsheet: CAPRISA 004 Trial and the impact of tenofovir gel on herpes simplex virus type-2 infections", , 20 July 2010 (2010-07-20), XP002649381, Retrieved from the Internet: URL:http://www.caprisa.org/joomla/Micro/CA PRISA%20004%20HSV-2%20factsheet_20%20July% 202010.pdf [retrieved on 2011-07-07]
- BALZARINI J ET AL: "Differential antiherpesvirus and antiretrovirus effects of the (S) and (R) enantiomers of acyclic nucleoside phosphonates: Potent and selective in vitro and in vivo antiretrovirus activities of (R)-9-(2-phosphonomethoxypropyl)-2,6-diami nopurine", ANTIMICROBIAL AGENTS AND CHEMOTHERAPY, vol. 37, no. 2, 1993, pages 332-338, XP002649382, ISSN: 0066-4804

## Description

### FIELD OF THE INVENTION

The invention relates generally to formulations of compounds with antiviral activity, for use in the prevention of acquisition and transmission of herpes simplex virus (HSV-1 and HSV-2), in particular HSV-2.

### BACKGROUND OF THE INVENTION

Human immunodeficiency virus (HIV) infection and related diseases are a major public health problem worldwide. Although great strides have been made in the prolongation of the life of AIDS patients by treatment with antiviral agents, there is no absolute cure.

Herpes Simplex Virus 2 (HSV-2) is another disease that is a major public health problem worldwide. It is estimated that HSV-2 is present in 20% of sexually active adults worldwide, and in men and women burdened with HIV prevalence of HSV-2 infection can rise to 80%. It is the most common cause of genital ulcer disease, but is almost entirely asymptomatic.

It has been demonstrated that there is a substantial link between the epidemics of sexually transmitted HIV and HSV-2, in that the presence of HSV-2 facilitates the acquisition of HIV. See, for example, "The Effects of Herpes Simplex Virus-2 on HIV acquisition and Transmission", J. Acquir. Immune Defic. Syndr., 2004; 35(5), by Lawrence Corey et al., in which it is disclosed that more than 30 epidemiologic studies have demonstrated that prevalent HSV-2 is associated with a 2- to 4-fold increased risk of HIV transmission and acquisition.

Accordingly, one approach to the problem of acquisition of HIV/AIDS and related diseases would be to reduce the risk of transmission of HIV and HSV-2, in order to reduce the number of individuals who become newly infected. Also, given that in many countries women are disproportionally affected by HIV infection as compared to men, (transmission of HIV from men to women is estimated to have a 7-fold greater efficiency as compared to women to men), a preferred approach would be to provide compositions that are effective against HIV and HSV-2 and can be used by women with or without their partners consent or knowledge.

[2-(6-Amino-purin-9-yl)-1-methyl-ethoxymethyl]-phosphonic acid (tenofovir, PMPA) is a well known compound with potent antiretroviral activity, useful in the treatment of patients with AIDS. See, for example, WO2006/017044, in which a tenofovir gel formulation is disclosed. The results of a recent clinical trial conducted in South Africa with this tenofovir gel formulation confirm the proposition that the formulation is effective in the prevention of transmission of HIV (Abdool Karim et al., Science 2010; 329: 1168-1174), and also demonstrated that the same tenofovir gel formulation is effective in hindering and/or preventing the transmission of HSV-2.. This is surprising, given that although previous publications have indicated that tenofovir is known to be a potent antiretroviral agent, it was not previously known to be effective as an anti-herpetic agent (see, for example, "Differential Antiherpesvirus and Antiretrovirus effects of the (S) and (R) Enantiomers of Acyclic Nucleoside Phosphonates", Antimicrobial Agents and Chemotherapy, Feb. 1993, p312-338, by J. Balzarini et al., Noesans et al., Antiviral Chemistry and Chemotherapy 8(1), p1-23 (1977), and De Clerq et al., Clinical Microbial Reviews, Vol. 16, No 4, p569-596 (2003).

### SUMMARY OF THE INVENTION

The present invention relates to formulations of compounds with antiviral activity, in particular [2-(6-amino-purin-9-yl)-1-methyl-ethoxymethyl]-phosphonic acid (tenofovir, PMPA), suitable for topical (e.g. vaginal, rectal, etc.) application and their use in hindering and/or preventing acquisition and transmission of HSV-2 infections.

In this invention, the formulation is a gel that is applied vaginally to a human female. The gel is applied before sexual activity, or after sexual activity, or before and after sexual activity, once or twice daily. One embodiment of the gel formulation comprises a mixture of tenofovir, hydroxyethylcellulose, propylparaben, methylparaben, edetate disodium, glycerin, citric acid, and purified water to 100%, with the addition of a small amount of 10% w/w sodium hydroxide and 10% w/w dilute hydrochloric acid to adjust the pH to about 4.4. In one example the gel formulation comprises:

| Ingredient | (%w/w |
|---|---|
| Tenofovir | 0.2-2.00 |
| Hydroxyethylcellulose, NF (Natrasol®250H) | 1-5.0 |
| Propylparaben, NF | 0.01-0.10 |
| Methylparaben, NF | 0.1-0.25 |
| Edetate Disodium, USP | 0.02-0.10 |
| Glycerin, USP | 3.00-30.00 |
| Citric Acid, USP | 0.5-2.00 |
| Purified Water, USP | to 100% |

with the addition of a small amount of 10% w/w sodium hydroxide and 10% w/w dilute hydrochloric acid to adjust the pH to about 4.4.

One embodiment of the gel formulation comprises:

| | %w/w |
|---|---|
| Tenofovir | 1.00 |
| Hydroxyethylcellulose, NF (Natrasol®250H) | 2.50 |
| Propylparaben, NF | 0.02 |
| Methylparaben, NF | 0.18 |
| Edetate Disodium, USP | 0.05 |
| Glycerin, USP | 20.00 |
| Citric Acid, USP | 1.00 |
| Purified Water, USP | 75.25 |
| Total | 100.00 |

with the addition of a small amount of 10% w/w sodium hydroxide and 10% w/w dilute hydrochloric acid to adjust the pH to a target of 4.4.

A second embodiment of the gel formulation comprises:

| | |
|---|---|
| | %w/w |
| Tenofovir | 1.00 |
| Hydroxyethylcellulose, NF (Natrasol®250H) | 3.0 |
| Propylparaben, NF | 0.005 |
| Methylparaben, NF | 0.22 |
| Edetate Disodium, USP | 0.05 |
| Glycerin, USP | 5.0 |
| Citric Acid, USP | 1.00 |
| Purified Water, USP | 89.66 |
| Total | 100.00 |

with the addition of a small amount of 10% w/w sodium hydroxide and 10% w/w dilute hydrochloric acid to adjust the pH to a target of 4.4.

A third embodiment of the gel formulation comprises:

| | %w/w |
|---|---|
| Tenofovir | 1.00 |
| Hydroxyethylcellulose, NF (Natrasol®250H) | 3.25 |
| Propylparaben, NF | 0.005 |
| Methylparaben, NF | 0.22 |
| Edetate Disodium, USP | 0.05 |
| Glycerin, USP | 5.0 |
| Citric Acid, USP | 1.00 |
| Purified Water, USP | 89.41 |
| Total | 100.00 |

with the addition of a small amount of 10% w/w sodium hydroxide and 10% w/w dilute hydrochloric acid to adjust the pH to a target of 4.4.

A fourth embodiment of the gel formulation comprises:

| | %w/w |
|---|---|
| Tenofovir | 1.00 |
| Hydroxyethylcellulose, NF (Natrasol®250H) | 3.5 |
| Propylparaben, NF | 0.005 |
| Methylparaben, NF | 0.22 |
| Edetate Disodium, USP | 0.05 |
| Glycerin, USP | 5.0 |
| Citric Acid, USP | 1.00 |
| Purified Water, USP | 89.16 |
| Total | 100.00 |

with the addition of a small amount of 10% w/w sodium hydroxide and 10% w/w dilute hydrochloric acid to adjust the pH to a target of 4.4.
Another embodiment of the invention relates to (R)-(1-(6-amino-9H-purin-9-yl)propan-2-yloxy)methylphosphonic acid for use in the reduction of or prevention of the transmission of HSV-2 to a mammal and for use in the reduction of or prevention of the acquisition of HSV-2 by a mammal., particularly where (R)-(1-(6-amino-9H-purin-9-yl)propan-2-yloxy)methylphosphonic acid is formulated as a gel, wherein the formulation comprises:

| | %w/w |
|---|---|
| Tenofovir | 1.00 |
| Hydroxyethylcellulose, NF (Natrasol®250H) | 2.50 |
| Propylparaben, NF | 0.02 |
| Methylparaben, NF | 0.18 |
| Edetate Disodium, USP | 0.05 |
| Glycerin, USP | 20.00 |
| Citric Acid, USP | 1.00 |
| Purified Water, USP | 75.25 |
| Total | 100.00 |

This invention generally relates to compositions and methods which prevent and/or reduce the risk of transmission of HSV-2 through sexual activity. Although it is mainly directed at heterosexual conduct (i.e., male/female vaginal intercourse), the compositions of this invention are also useful for parties engaged in other types of sexual behaviour. For example, the compositions of this invention could be used by parties engaged in anal intercourse (male/female or male/male); compositions of this invention intended to be used in anal intercourse are preferably modified to adjust the buffering capacity to pH values normally found in the rectum and by altering the lubricity of the formulation.

For vaginal heterosexual intercourse, the composition may be inserted into the vagina prior to intercourse. For anal intercourse (heterosexual or homosexual), the composition may be inserted into the rectum prior to intercourse. For either vaginal or anal intercourse, the composition may also act as a lubricant. For added protection it is generally preferred that the composition be applied-before intercourse or other sexual activity and that, if appropriate, a condom be used. For even further protection, the composition may be reapplied as soon as possible after completion of the sexual activity.

If desired, flavorings, scents, fragrances, and colorants may be incorporated into the composition so long as they do not interfere with the safety or efficacy of the composition. Indeed, incorporation of such flavorants, scents, fragrances, and colorants into the compositions of this invention may increase the probability that the composition will be used during sexual activity.

One advantage of the present method is that it can be used for protection during a wide variety of sexual activities (vaginal or anal) by heterosexuals, bisexuals, and homosexuals. Another advantage of the present method of reducing the transmission of HSV-2 is that this method may be implemented and/or used most easily by the party being penetrated. Thus, a woman may use the present method to protect herself (as well as her partner) with or without the partner's knowledge of the method being used. Moreover, the partner would not be required to rely on his or her partner's claim of being AIDS-free or agreement to use condoms for protection. Either or both sexual parties (especially the female participant) could initiate and implement the use of the present method. Preferably the method is used before the sexual activity and most preferably both before and after the sexual activity.

### DETAILED DESCRIPTION

Recently, instead of testing new compounds as potential microbicides, tenofovir, a highly selective and efficient nucleotide HIV reverse transcriptase (RT) inhibitor widely used in HIV therapy, was formulated as a 1% gel and tested in a double-blind placebo-controlled study with approximately 900 African women. The results of this trial showed an overall decrease of 39% in the frequency of the sexual transmission of HIV-1. This prophylactic effect further increased to a 54% reduction of transmission among women with high adherence to the tenofovir treatment. Thus, this trial became the first example of a microbicide convincingly diminishing HIV-1 transmission.

Surprisingly, a significant 51 % reduction of the risk of acquisition of herpes simplex virus type 2 (HSV-2) was also observed in the trial. This observation is important, since HSV-2 is a common copathogen with HIV-1 which facilitates HIV transmission, presumably by inducing genital ulceration and inflammation. This effect of tenofovir gel on HSV was unanticipated, since this highly potent anti-retroviral and anti-hepadnaviral drug has been previously shown to exhibit minimal, if any, in vitro activity against HSV and most of the other DNA viruses.

The present study provides evidence that at the concentrations achieved intravaginally by the topical administration of a 1% gel of tenofovir are equivalent to those that inhibit the replication of both HSV-1 and HSV-2 in multiple cell lines and primary cell types. Thus, the anti-herpetic activity of tenofovir observed in the clinical trial is a result of its direct anti-herpetic effect.

Reference will now be made in detail to certain embodiments of the invention. While the invention will be described in conjunction with the enumerated embodiments, it will be understood that they are not intended to limit the invention to those embodiments. On the contrary, the invention is intended to cover all alternatives, modifications, and equivalents, which may be included within the scope of the present invention as defined by the claims.

### DEFINITIONS

Unless stated otherwise, the following terms and phrases as used herein are intended to have the following meanings:

The term "therapeutically-effective amount" refers to an amount of a compound that, when administered to a subject for treating a disease, is sufficient to effect treatment for the disease. "Therapeutically effective amount" can vary depending on the compound, the disease and its severity, the age, the weight, etc. of the subject to be treated.

"Bioavailability" is the degree to which the pharmaceutically active agent becomes available to the target tissue after the agent's introduction into the body. Enhancement of the bioavailability of a pharmaceutically active agent can provide a more efficient and effective treatment for patients because, for a given dose, more of the pharmaceutically active agent will be available at the targeted tissue sites.

The compounds of the combinations of the invention may be referred to as "active ingredients" or "pharmaceutically active agents."

PMPA or tenofovir (US Patent Nos. 4,808,716, 5,733,788, 6,057,305) has the structure:

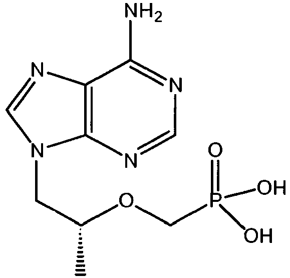

The chemical names of PMPA, tenofovir include: (R)-9-(2-phosphonylmethoxypropyl)adenine; (R)-(1-(6-amino-9H-purin-9-yl)propan-2-yloxy)methylphosphonic acid; and phosphonic acid, [[(1R)-2-(6-amino-9H-purin-9-yl)-1-methylethoxy]methyl]. The CAS Registry number is 147127-20-6.

Tenofovir diphosphate has the structure: 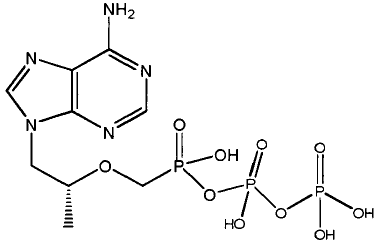 and is named (R)-(1-(6-amino-9H-purin-9-yl)propan-2-yloxy)methylphosphonic diphosphoric anhydride.

Also disclosed is topical administration of the formulation to the anus. Also disclosed is oral application which is suitably carried out by applying a composition which is in the form of a mouthwash or gargle. Oral application is especially preferred to prevent infection during dental procedures. Suitably, the composition is applied just prior to the beginning of the dental procedure and periodically throughout the procedure.

The present invention also includes formulations that are aerosols, foams, jellies, creams, suppositories, tablets, tampons, etc., and the use of a condom which is coated with the formulation. In a preferred embodiment, the condom is coated with a lubricant or penetration enhancing agent that comprises an antiviral compound, preferably tenofovir. Lubricants and penetration enhancing agents are described in U.S. Patent Nos. 4,537,776; 4,552,872; 4,557,934; 4,130,667, 3,989,816; 4,017,641; 4,954,487; 5,208,031; and 4,499,154.

### EXAMPLES

The following examples further describe and demonstrate particular embodiments within the scope of the present invention. The examples are given solely for illustration and are not to be construed as limitations as many variations are possible without departing from the scope of the claims. The following examples are intended for illustration only and are not intended to limit the scope of the invention in any way. "Active ingredient" denotes one or more NRTIs, as defined above, preferably tenofovir or a physiologically functional derivative thereof

### MATERIALS AND METHODS

Cells. Human embryonic lung (HEL) fibroblasts (HEL-299; ATCC CCL-137) were cultured in MEM Earle's medium (Gibco, Invitrogen Corporation, UK) supplemented with 10% fetal calf serum (FCS), 1% L-glutamine, 1% non-essential amino acids and 1% sodium pyruvate. Primary human keratinocytes (PHKs) were isolated from neonatal foreskins. Tissue fragments were incubated with trypsin-EDTA for 1 h at 37°C. The epithelial cells were detached and cultured in Keratinocyte Serum-Free Medium (Keratinocyte-SFM), (Gibco, Invitrogen Corporation, UK) containing the following supplements: 0.5 µg of hydrocortisone per ml, 10 ng of epidermal growth factor per ml, 2 mM of L-glutamine, 10 mM of HEPES, 1 mM of sodium pyruvate, 1 x 10⁻¹⁰ M of cholera toxin, 5 µg of insulin per ml, 5 µg of human transferrin per ml and 15 x 10⁻⁴ mg of 3,3',5-triiodo-2-thyronine per ml. The PHKs were used for the antiviral assays in monolayers and for preparation of organotypic raft cultures. The TZM-bl cells were kindly provided by Dr. G. Van Ham (ITG, Antwerp, Belgium).

Viruses. The HSV-1 strains KOS and F and the HSV-2 strains G and MS were used as reference herpes viruses. Several HSV-1 wild-type (wt) [RV-6, RV-132, RV-134, C559143], HSV-1 thymidine kinase-deficient (TK⁻) [RV-36, RV-117, 328058], HSV-2 wt [RV-24, RV-124,NA, PB, NS, HSV-47] and HSV-2 TK⁻ [RV-101, RV-129, 19026589, LU, HSV-44] clinical strains isolated from virus-infected individuals in Belgium were used. HIV-1 strain III_{B} was provided by R.C. Gallo (at that time at the National Institutes of Health, Bethesda, MD).

Compounds. The sources of compounds were as follows: acyclovir [ACV, 9-(2-hydroxyethoxymethyl)guanine], GlaxoSmithKline, Stevenage, UK; ganciclovir [GCV, 9-(1,3-dihydroxy-2-propoxymethyl)guanine, Roche, Basel, Switzerland; penciclovir [PCV, 9-(4-hydroxy-3-hydroxymethylbut-1-yl)guanine], Aventis, Frankfurt, Germany; brivudin [(E)-5-(2-bromovinyl)-1(-D-2'-deoxyribofuranos-1-yl-uracil, BVDU], Searle, UK; (S)-HPMPC [cidofovir, CDV, (S)-1-(3-hydroxy-2-phosphonylmethoxypropyl)cytosine], PMEA, [adefovir, ADV, 9-[2-(phophonylmethoxyethyl)adenine] and (R)-PMPA [tenofovir, TFV, (R)-9-[2-(phophonylmethoxypropyl)adenine]] Gilead Sciences, Foster City, CA. Tenofovir diphosphate (TFV-DP) and acyclovir triphosphate (ACV-TP) were obtained from Moravek Biochemicals, Brea, CA.

Radiochemicals. [³H]tenofovir (radiospecificity: 15 Ci/mmol), [8-³H]dGTP (radiospecificity: 17.9 Ci/mmol) and [2,8-³H]dATP ((radiospecificity: 153 Ci/mmol) were from Moravek Biochemicals (Brea, CA).

HSV cytopathic effect (CPE) reduction assay. HEL and PHK cells were used to perform the CPE reduction assay. Both cell types were cultured in 96-well microtiter plates in their corresponding growth medium. Confluent monolayers were infected with each viral strain at 100 CCID₅₀ (1 CCID₅₀ corresponds to the virus stock dilution that is infective for 50% of the cell cultures). The medium used to allow viral infection and growth in HEL cells was MEM Earle's medium containing 2% FCS. After a 2-h adsorption period, residual virus was removed and the infected cells were further incubated in medium containing serial dilutions of the test compounds (in duplicate). After 2 to 3 days of incubation time, viral cytopathicity (CPE) was visually assessed, and the 50% effective concentration [EC₅₀, compound concentration required to reduce viral CPE by 50%] was determined. The EC₅₀s of the compounds tested against each viral strain were calculated as the mean values of at least two independent experiments. The assays were performed in a similar way in PHKs, except that Dulbecco-F12 medium [a mixture of 1/3 HAM F12 and 2/3 Dulbecco's modified Eagle's medium, supplemented with 10% fetal calf serum (FCS), 2 mM L-glutamine, 10 mM HEPES, 1 mM sodium pyruvate, 5 ml of 100× antibiotic-antimycotic per liter (Gibco, Invitrogen Corporation)] used for viral infection and a 50/50 (v/v) mixture of Keratinocyte-SFM and Dulbecco-F12 medium was added following viral adsorption.

Herpes virus infection of primary monocyte/macrophage cell cultures. Human peripheral blood mononuclear cells (PBMCs) were obtained from the blood of healthy seronegative donors by Ficoll-Hypaque density gradient centrifugation. The PBMCs were resuspended in RPMI 1640 medium supplemented with 20% heat-inactivated (56°C, 30 min) fetal calf serum (FCS), penicillin (100 U/mL), streptomycin (100 mg/L) and L-glutamine (2 mM), then seeded into 48-well plates (1.8×10⁶ cells/well). Monocyte/macrophage (M/M) cells were separated by adherence onto plastic. After 5 days, non-adherent cells were carefully removed by repeated gentle washings with warm medium, and adherent (95% pure) M/M were cultured for an additional 3 days to mature and to form a monolayer. African green monkey fibroblastoid kidney (Vero) cells highly sensitive to the cytopathic effect of HSV-2 were grown in RPMI medium supplemented with 10% heat inactivated FCS and were used in infectious virus titration assays. To evaluate the anti-HSV2 activity of tenofovir in human macrophages , the compound was added to macrophages 1 hour before infection at increasing concentrations (0.04, 0.2, 1, 5, 20, 100 or 500 µg/ml). Similarly, macrophage cultures were treated with different concentrations of adefovir (0.04, 0.2, 1, 5, 20 or 100 µg/ml) or acyclovir (0.008, 0.04, 0.2, 1, 5, 20 or 100 µg/ml) used as a control drug.

Macrophage cultures were then infected with HSV-2 (100 CCID₅₀) in the presence of the test compounds. After 2 hrs virus adsorption, the cultures were extensively washed to remove any residual virus particles. Fresh culture medium and compounds, at the indicated concentrations, were then added to the cultures. Tenofovir, adefovir and acyclovir were maintained throughout the experiment. Appropriate positive (infected but not treated M/M) and mock-infected negative (uninfected and untreated M/M) controls were run for each experiment as well. All assays were performed in triplicate. The cytopathic effect on macrophages was daily monitored by microscopic observation and reached completion at day 5-6 post infection. Therefore, the potential inhibitory effect of the compounds on the replication of HSV-2 was evaluated 6 days after infection. The amount of infectious virus in the supernatants was determined by a classical limited dilution assay on Vero cell cultures. The titers of produced virus were calculated according to the Reed and Muench method and expressed as 50% tissue culture infective dose per ml (TCID₅₀/ml).
The inhibition capacity was expressed in % and calculated considering as value 100 being the virus production in virus-infected untreated cultures.

Co-infection of TZM-bl cells with HIV-1 and HSV-2. The TZM-bl cell line is derived from HeLa cells and expresses high levels of CD4, CCR5 and CXCR4. In addition, this cell line is stably transduced with a LTR-driven firefly luciferase and E.coli -galactosidase gene (15). In a 96-well tray, 10,000 TZM-bl cells were seeded on day 1. On day 2, the supernatant was aspirated and 100 µl of serial dilutions of tenofovir was added to the cell cultures. Next, 100µl of a virus suspension containing either HIV-1 (NL4.3), HSV-2 (G) or both viruses together was administered. Two to three days post infection, HIV-1 infection was monitored based on the determination of luciferase activity. For this means, 100 µl of culture supernatant was removed and 100 µl of Bright-Glo Luciferase Assay Substrate (Promega, Madison, USA) was added. The luminescence signal was measured using the Safire 2 microtiter plate reader (Tecan, Mannedorf, Switzerland). A control condition containing only mock-infected cells was also included to measure the background luminescence levels. Three days post infection, HSV-2-induced cytopathicity was recorded microscopically based on giant cell formation.

Organotypic epithelial raft cultures. For the preparation of epidermal equivalents, a collagen matrix solution was made with collagen mixed on ice with 10-fold concentrated HAM's F12 media, 10-fold reconstitution buffer and Swiss 3T3 J2 fibroblasts. One milliliter of the collagen matrix solution was poured into the wells of 24-well microtiter plates. After gel equilibration with 1 ml of growth medium overnight at 37 °C, 2.5 x 10⁵ PHK cells were seeded on the top of the gels and maintained submerged for 24-48 hours. The collagen rafts were raised and placed onto stainless-steel grids at the interface between air and liquid culture medium. The growth medium was a mixture of 1/3 HAM F 12 and 2/3 Dulbecco's modified Eagle's medium, with the same supplements as used for the Keratinocyte-SFM. Epithelial cells were allowed to stratify, the medium being replaced every 2-3 days. For the evaluation of the antiviral effects of the compounds in the raft system, two series of rafts were run in parallel, one for histological examination and the other one for quantification of viral production by a plaque reduction assay. Rafts were infected with 5,000 PFU of HSV-1 (KOS) or HIV-2 (G) strains after 10 days post-lifting and then the medium was replaced by medium containing different dilutions of the compounds. The growth medium containing the different concentrations of the compounds was changed two days later and 3 days later (after 15 days of differentiation), one series of rafts was fixed in 10% buffered formalin, embedded in paraffin and stained with hematoxylin and eosin for histological evaluation. Another series of rafts was used to quantify virus production. For that purpose, each raft was frozen in 3 ml phosphate buffer saline (PBS) and thawed to release the virus from the infected epithelium. Supernatants were clarified by centrifugation at 1,800 rpm and titrated by a plaque assay in HEL cell cultures. Virus production in each raft was then calculated. Two rafts were used for each drug concentration to determine the effects of the compounds on virus yield.

Human ex vivo tissues. Human tonsillar tissues were obtained from patients undergoing routine tonsillectomy at the Children's National Medical Center (Washington, DC) under IRB-approved protocol. Cervical tissues were obtained through the National Disease Research Interchange (NDRI, Philadelphia, PA). Tissues were dissected into about 8-mm³ blocks and placed onto collagen sponge gels in culture medium at the air-liquid interface, as described earlier (16). Briefly, tissue blocks were cultured in RPMI 1640 (GIBCO BRL, Grand Island, NY) medium containing 15% heat-inactivated fetal calf serum (FCS; Gemini Bio-Products, Woodland, CA).
Tonsillar tissue: For each experimental condition, 27 tissue blocks (9 blocks/well/3 ml of complete medium) were inoculated with 5 µL of viral stock of HSV-1 (strain F) or HSV-2 (strains G and MS) (ATCC) placed on top of each tissue block. Coinfection experiments were performed by inoculating tissue blocks sequentially with 5 µL of HSV-2 (strain G) and 5 µL (0.5 ng of p24) of X4_{LAI.04} (obtained from the Rush University Virology Quality Assurance Laboratory (Chicago, IL)). In all experiments using tonsillar tissues, tenofovir was added to the culture medium 12h prior to viral infection and replenished at each culture medium change.
Cervico-vaginal tissue: For each experimental condition 16 tissue blocks were immersed in 500 µl of a HSV-2 (strain G) suspension for 2 hours at 37°C, washed three times with PBS and then placed on the gelfoam rafts. Tenofovir was added during the infection and replenished at each culture medium change. Herpes simplex viral replication was evaluated by the release of viral DNA into the culture medium as measured by quantitative real-time PCR (17). HIV-1 replication was evaluated by the release of p24 capsid antigen using a bead-based assay (18).

In vivo antiviral activity of HSV-1 and HSV-2-infected mice. Adult NMRI athymic nude mice (weighing ∼20 g) were scarified on the lumbosacral area over a surface of about 1 cm². Mice were inoculated with 5x10³ PFU of HSV-1 (Kos strain) or 5x10² of HSV-2 (G strain) in 50 µL per mouse. Topical 1% formulations of tenofovir, adefovir, and cidofovir were prepared in 100% dimethylsulfoxide or in a gel identical to that used in the CAPRISA 004 trial. In each experiment, a group of animals treated with a placebo formulation that was the same as the test formulation without drug was included as a negative control. In the treatment protocol, animals were administered tenofovir, adefovir, or cidofovir topically at the indicated formulation and drug concentration twice a day for a period of five days starting 1-2 h after infection. The day of virus inoculation was always considered as day 0. All animal procedures were approved by the K.U.Leuven Animal Care Committee. Development of lesions and mortality were recorded over a one month period. Animals were euthanized when more than 30% loss in body weight or development of paralysis occurred. Survival rates were estimated according to the Kaplan-Meir method and were compared using the log-rank test (Mantel-Cox) test (GraphPad Prism).

Metabolism of tenofovir in lymphocyte CEM, fibroblast HEL and epithelial TZM-bl cell cultures. The metabolism of radiolabeled tenofovir was monitored as follows: CEM, HEL or TZM-bl cells were seeded at 4 x 10⁵, 5.1 x 10⁵ and 17 x 10⁵ cells/ml, respectively, in 5-ml culture flasks (25 cm²) and incubated with 2 µM [2,8-³H]tenofovir (10 µCi/flask). The radiolabeled drug was added to the cell cultures for 24 hrs at 72 hrs after the time of seeding of the cells. At this time point, the cells were centrifuged (after prior detachment from the culture recipient for HEL and TZM-bl cells) at 4°C, thoroughly washed twice with ice-cold medium (without serum) and precipitated with 60% cold methanol. After centrifugation at 10,000 rpm, radiolabeled [³H]tenofovir and its metabolites in the supernatants were quantified by HPLC analysis using a Partisil-SAX-10 radial compression column as previously described (19). The retention times for [³H]tenofovir, [³H]tenofovir-MP and tenofovir-DP were approximately at 5, 16 and 32 min.

HSV-1 DNA polymerase and HIV-1 reverse transcriptase assay. The reaction mixture (40 µl) for the HSV-1 DNA polymerase and HIV-1 RT assays contained 4 µl Premix (200 mM Tris.HCl, pH 7.5; 2 mM DTT; 30 mM MgCl₂), 4 µl BSA (5 mg/ml), 1.6 µl activated calf thymus DNA (1.25 mg/ml), 0.8 µl dCTP (5 mM), 0.8 µl dTTP (5 mM), 0.8 µl dGTP (5 mM), 2 µl radiolabeled [³H]dATP (1 mCi/ml) (3.3 µM), 18 µl H₂O and 4 µl tenofovir-DP at different concentrations (i.e. 200, 20, 2, 0.2 µM). The reaction was started by the addition of 4 µl recombinant HSV-1 DNA polymerase (kindly provided by M.W. Wathen (Pfizer, Kalamazoo, MI)) or recombinant HIV-1 RT (in 20 mM Tris.HCl, pH 8.0; 1 mM DTT; 0.1 mM EDTA; 0.2 M NaCl; 40% glycerol), and the reaction mixture was incubated for 60 min (HSV-1 DNA polymerase) or 30 min (HIV-1 RT) at 37°C. Then, 1 ml ice-cold 5% TCA in 0.02 M Na₄P₂O₇.10 H₂O was added to terminate the polymerisation reaction, after which the acid-insoluble precipitate (radiolabeled DNA) was captured onto Whatman glass fiber filters type GF/C (GE Healthcare UK Limited, Buckinghamshire, UK) and further washed with 5% TCA and ethanol to remove free radiolabeled dATP. Radioactivity was determined in a Perkin Elmer Tri-Carb 2810 TR liquid scintillation counter.

### RESULTS

### Inhibition of HSV-1 and HSV-2 replication by Tenofovir in multiple permissive cell types

The activity of tenofovir against laboratory HSV strains was first evaluated in HEL cell monolayers and primary human keratinocytes (PHKs) and compared with the anti-HSV activity of nucleoside (i.e. acyclovir, penciclovir, ganciclovir, and brivudin) and acyclic nucleotide phosphonate (ANP) (i.e. cidofovir and adefovir) analogues. Tenofovir inhibited virus-induced cytopathicity with EC₅₀ values of ∼ 100 to 200 µg/ml against HSV-1 and HSV-2 in PHKs. EC₅₀ values for adefovir were 3.6 to 13 µg/ml and for cidofovir 0.63 to 4.4 µg/ml. Except for brivudin, which is known to have a markedly lower activity against HSV-2 than HSV-1, nucleoside analogs proved more active than any of the acyclic nucleotide phosphonate analogs tested.

Tenofovir was also evaluated side-by-side with the acyclic nucleoside phosphonates (ANPs) adefovir and cidofovir, and with several nucleoside analogs against a variety of HSV-1 and HSV-2 clinical isolates, including wild-type and acyclovir-resistant virus strains in HEL fibroblast cell cultures. Tenofovir inhibited the cytopathic effects of all clinical isolates with mean EC₅₀ values of 130 µg/ml (range of 114-160 µg/ml), ≥ 166 µg/ml (range of 117 - ≥ 200 µg/ml), ≥ 157 µg/ml (range of 125- ≥200 µg/ml), and 152 µg/ml (range of 131-179 µg/ml) against, respectively, HSV-1 wt, thymidine kinase-deficient HSV-1 TK⁻, HSV-2 wt, and thymidine kinase-deficient HSV-2 TK. When tested in parallel, adefovir showed mean EC₅₀ values that were 20- to 32-fold lower than those seen for tenofovir whereas cidofovir was the most active ANP with mean EC₅₀ values ranging between 0.35 µg/ml and 0.67 µg/ml. Thus, the antiherpetic EC₅₀ values for tenofovir were 181- to 474-fold higher than for cidofovir. In contrast to the tested ANPs that showed similar EC₅₀ values for wild-type and mutant TK- HSV clinical isolates, acyclovir, ganciclovir, penciclovir and brivudin lost their antiherpetic activity against the thymidine kinase-deficient herpes virus strains.

Tenofovir, adefovir and acyclovir have also been evaluated for their anti-HSV-2 activity in primary monocyte/macrophage (M/M) cell cultures. Herpes virus production amounted up to 2.8 ± 0.9 x 10⁵ TCID₅₀/ml in the supernatants of the control virus-infected cultures, resulting in a microscopically visible 90-100% cytopathic effect (CPE) (Fig. 1). Tenofovir at the concentrations of both 500 and 100 µg/ml completely suppressed virus replication without any sign of microscopically visible CPE at day 6 post virus infection. At 20 and 5 µg/ml, the virus titers in the culture supernatants were more than one log lower than in untreated controls resulting in ∼ 5% and 30% visible CPE, respectively. At 1 µg/ml or 0.2 µg/ml drug concentrations, no pronounced protective effect of tenofovir was observed (2.5 x 10⁵ TCID₅₀/ml; ≥ 70-80% CPE) (Fig. 1). As expected, adefovir was more antivirally active in M/M. At drug concentrations between 500 and 5 µg/ml, neither viral particles in the supernatants nor visible CPE were found in the cell cultures. Even adefovir concentrations as low as 1 and 0.4 µg/ml markedly reduced HSV titers (8.5 x 10² and 5.4 x 10³ TCID₅₀/ml) and the visible CPE was 5% and 45%, respectively. As expected, the established antiherpetic drug acyclovir proved extremely efficient in inhibiting herpes virus replication in the M/M cell cultures (full suppression of virus released in the supernatants and no visible cytopathicity at concentrations equal to, or higher than 0.008 µg/ml).

### Inhibition of HSV-2 replication by Tenofovir in HIV-infected epithelial TZM-bl cell cultures

Epithelial TZM-Bl cells are derived from HeLa cells transfected with the HIV (co)-receptors CD4, CXCR4 and CCR5. They can be infected both by HIV and HSV. We co-infected these cells with HIV-1(NL4.3) and HSV-2(G) and monitored HIV replication by the LTR-driven luciferase expression (luminescence), and HSV-2 replication by microscopic reading of virus-induced cytopathicity. Tenofovir prevented HSV-2 infection in co-infected cultures at a drug concentration of ∼60 µg/ml, which is similar to the tenofovir concentration required to inhibit singly HSV-2-infected TZM-bl cell cultures. Also, HIV-1 was equally suppressed by tenofovir in the presence or absence of an ongoing HSV-2 infection (data not shown). Thus, under the experimental conditions of dual HIV-1 and HSV-2 infection, each virus did not affect the antiviral activity of tenofovir against the other virus.

### Suppression of viral replication by Tenofovir in organotypic epithelial raft cultures

As differentiated keratinocytes are the main target cells for productive infection of HSV in vivo, the antiviral activity of tenofovir in organotypic raft cultures of keratinocytes was evaluated. In this three-dimensional culture model, keratinocytes fully differentiate, thus faithfully resembling the in vivo tissue status. We compared in this system the ability of tenofovir, adefovir and cidofovir to reduce replication of HSV. The organotypic epithelial raft cultures were infected after 10 days of differentiation and treated with serial dilutions of the test compounds. Fifteen days post-lifting (i.e. after 5 days of treatment), the rafts were processed for histological examination and for viral quantification. Histological examination of the culture sections showed a completely differentiated epithelium with characteristic layers in control rafts while HSV-infected rafts showed pronounced viral infection and viral spread all along the epithelium (Fig. 2). Infection of the rafts with HSV produced cytopathic effects resulting in ballooning and reticular degeneration of the keratinocytes together with the occurrence of intranuclear eosinophilic inclusion bodies, formation of typical intraepithelial vesicles and multinucleation.
Morphological analysis of the organotypic cultures showed that treatment with tenofovir at 200 µg/ml and 50 µg/ml protected the entire epithelium against HSV-2-induced cytopathicity, while at 20 µg/ml and 5 µg/ml, the compound was partially protective, with areas of a normal epithelium and areas with destructed rafts (Fig. 2). At a concentration of 2 µg/ml tenofovir was inactive against HSV-2. Administration of adefovir at ≥ 2 µg/ml and cidofovir at 0.2 µg/ml resulted in complete protection of the epithelial tissue (data not shown). No toxic effects, measured as an increase in the number of dead cells or alteration of differentiation, were observed at the highest concentration of all the tested compounds (200 µg/ml for tenofovir, and 50 µg/ml for adefovir and cidofovir). The selective anti-HSV effect of tenofovir, adefovir, and cidofovir was also confirmed when the raft cultures were infected with a reference HSV-1 laboratory strain. In this case, complete protection from virus-induced cytopathic effect was seen at a concentration of 200 µg/ml of tenofovir, 2 µg/ml of adefovir, and ≥ 0.5 µg/ml of cidofovir. Lower concentrations of the compounds resulted in partial protection (i.e. 50 µg/ml of tenofovir) or full destruction (i.e. 20 µg/ml of tenofovir) of the epithelium.
In order to quantify the antiviral effects of tenofovir compared to adefovir and cidofovir, the virus yield per raft was determined by quantitative PCR. As shown in Fig. 3, a concentration-dependent inhibition of viral production per raft was observed following treatment of the rafts with serial concentrations of the compounds. Tenofovir demonstrated a 2.6- (HSV-1) and a 5.4- (HSV-2) log reduction in virus production (plaque forming units (PFU)) at the highest concentration tested (i.e. 200 µg/ml). At a concentration of tenofovir at 50 µg/ml, a reduction in virus yield by 0.81 (HSV-1) and 1.75 (HSV-2) logs was observed. Even at 20 µg/ml, a 0.9 log reduction in virus production was observed in rafts infected with HSV-2. Lower concentrations of the compound were unable to reduce virus replication in the rafts. As expected, both adefovir and cidofovir proved more active against HSV replication than tenofovir (Fig. 3A and 3B).

### Suppression of Herpes Simplex virus type 2 by Tenofovir in singly infected and in HIV-1 coinfected human ex vivo lymphoid tissue

The effect of tenofovir on the replication of HSV-1 strain F (HSV-1_{F}), HSV-2 strain G (HSV-1_{G}) and HSV-2 strain MS (HSV-2_{MS}) was investigated in infected human tonsillar tissues ex vivo (Fig. 4). This system supports replication of various viruses (17) without exogenous stimulation or activation. Upon inoculation in this in vivo-like tissue system, HSV-1_{F}, HSV-2_{G} or HSV-2_{MS} efficiently replicated as shown by the presence of viral DNA in culture medium bathing the tissue blocks. Tissues from all the tested donors supported a pronounced productive viral infection with a median accumulation of the viral DNA genome in the culture medium throughout the 9 days of culture reaching 7.8 log₁₀ DNA copies/ml [Interquartile range (IQR) 7.1-8.1, n=5], 7.25 log₁₀ copies/ml (IQR 7.2-7.9, n=6) and 6.4 log₁₀ copies/ml (IQR 6.1-7.5, n=3) for HSV-1_{F}, HSV-2_{G} and HSV-2_{MS}, respectively.
To test the effect of tenofovir on HSV replication, blocks of human tonsillar tissues were treated overnight with drug concentrations ranging from 3 to 240 µg/ml and infected with HSV-1_{F}, HSV-2_{G} or HSV-2_{MS}. Tenofovir was maintained throughout the entire culture period and replaced with each medium change. Tenofovir suppressed the replication of HSV-1_{F}, HSV-2_{G} and HSV-2_{MS} in a dose-dependent manner with an EC₅₀ of 7 µg/ml [95% Confidence Interval (CI):10-44] for HSV-1_{F}; 14 µg/ml (CI: 10-163) for HSV-2_{G} (Fig. 4A), and 19 µg/ml (CI: 27-127) for HSV-2_{MS}. Accordingly, tenofovir at the concentration of 66 µg/ml reduced HSV-1_{F}, HSV-2_{G} and HSV-2_{MS} replication by respectively 99 ± 0.1%, 94 ± 7% (Fig. 4A) and 89 ± 2% compared to infected donor matched-untreated tissue (p<0.01). At day 9 post infection in tissues treated with 66 µg/ml tenofovir, HSV-1_{F} replication as measured by the release of viral DNA in culture medium was 4 log₁₀ DNA copies/ml (IQR 3.9 - 4.3) compared to 8 log₁₀ DNA copies/ml (IQR 7.87 - 8.04) in untreated matched donor tissue (n=3). Similarly, at day 9 post infection, HSV-2_{G} and HSV-2_{MS} replication resulted in 7.6 log₁₀ DNA copies/ml (IQR 7.4 - 7.8) and 7.3 log₁₀ DNA copies/ml (IQR 6.9 - 7.6), respectively, in untreated tissues, while it was 5.2 log₁₀ DNA copies/ml (IQR 3.6 - 6.1, n=14) and 5.8 log₁₀ DNA copies/ml (IQR 5.7-6.1, n=3) in matched donor tissues treated with 66 µg/ml tenofovir.
Even at the highest drug concentrations, the suppression of HSV-1_{F}, HSV-2_{G} and HSV-2_{MS} replication was not associated with measurable tonsillar tissue lymphocyte depletion: there was no statistically significant difference in the absolute number either of total T cells (CD3⁺), total B cells (CD19⁺) or subsets of naive and memory T-cells between tissues treated with 60 µg/ml tenofovir and donor-matched untreated tissues (n=3, p>0.4).
To evaluate the dual antiviral activity of tenofovir against HSV-2 and HIV-1, we coinfected tonsillar tissues with HSV-2_{G} and HIV-1_{X4LAI} as described earlier (16) and treated them with tenofovir at the concentration of 66 µg/ml throughout the entire culture period. In the untreated control tissues, HSV-2_{G} DNA release into culture medium was 7.3 log₁₀ copies/ml (IQR 6.8 - 7.4) while in donor-matched tenofovir-treated tissues coinfected with HIV-1, the HSV-2_{G} DNA release into culture medium was 5 log₁₀ copies/ml (IQR 4.3 - 5.7, n=6). Thus, in these tissues, 66 µg/ml tenofovir suppressed HSV-2_{G} replication by 96 ± 1% (n=6; p<0.01) (Fig. 4B). On day 9 post infection, HIV-1_{X4LAI} replication as measured by p24 release into the culture medium was fully suppressed in all the tested tissues (2462 ± 1117 pg/ml in untreated controls vs. 0 pg/ml in donor-matched tenofovir-treated tissues (Fig. 4B).
To confirm the specificity of the antiviral activity of tenofovir on HSV-2_{G}, we treated tissues with the potent HIV nucleoside reverse transcriptase inhibitor (NRTI) lamivudine at the concentration of 33 µg/ml. In tissues from two donors, we found no effect of lamivudine on HSV-2_{G} replication compared to untreated donor-matched HSV-2_{G}-infected tissues (data not shown). As expected, lamivudine has shown a potent effect on HIV-1 replication. Thus, the antiherpetic effect of tenofovir is not a general property of the NRTIs.
Also, to evaluate possible immunomodulatory effects of tenofovir treatment in ex vivo tonsillar tissues, we measured the concentration of 20 cytokines (IL-1, IL-1, IL-2, IL-6, IL-7, IL-8, IL-15, IL-16, IFN, CCL3/MIP-1, CCL4/MIP-1, CCL20/MIP-3, CCL5/RANTES, CXCL12/SDF-1, TGF, TNF, CCL2/MCP-1, CCL11/Eotaxin, CXCL9/MIG, CXCL10/IP-10') in culture medium from donor-matched tissues treated for 9 days with tenofovir at a concentration of 66 µg/ml. On day 9 post infection there were no significant differences between the concentrations of any of the evaluated cytokines in untreated tissues and tissues treated with tenofovir (p > 0.15).

### Suppression of HSV-2 by Tenofovir in human ex vivo cervico-vaginal tissues

To investigate the anti-HSV activity of tenofovir in cervico-vaginal tissues blocks of this tissue were cultured as described earlier, inoculated with HSV-2_{G} and treated with the drug. Tenofovir at the concentration of 166 µg/ml was added at the time of HSV-2_{G} infection and maintained throughout the duration of the experiment by replenishing it with each media change. HSV-2_{G} replication was evaluated by the release of viral DNA into the culture medium bathing the cervico-vaginal tissue blocks (16 blocks per condition). In control tissues not treated with tenofovir, viral replication was detected in tissues from all 5 tested donors with a median cumulative production of 6.6 log₁₀ copies/ml (IQR 5.3-8.2) throughout 12 days of culture. In cervico-vaginal tissues treated with tenofovir, viral production was reduced to 5.5 log₁₀ copies/mL (IQR 4.8 - 5.7, n=5) (Fig. 4C and Fig. 4D) reflecting 78 ± 9 % reduction when the reductions of viral replication in each experiment were averaged (p<0.01).

### Activity of tenofovir in HSV-infected mice

Tenofovir, adefovir, and cidofovir were evaluated as antiherpetics in HSV-1- and HSV-2-infected mice using two different vehicles - DMSO (Fig. 5) and a gel used in the CAPRISA 004 study (data not shown). All compounds were administered to HSV-1- and HSV-2-infected mice at a concentration of 1% for 5 days, starting at the day of infection. Both the morbidity and the survival curves of mice infected with HSV-1 and treated with 1 % tenofovir were significantly delayed compared to the placebo-treated group. As expected, adefovir proved to be more effective both in the DMSO (Fig. 5A) and gel formulations (data not shown) compared to tenofovir since 80% and 100% survival was observed when animals received 1% adefovir in DMSO or gel, respectively. It should be mentioned that the mice treated with 1% adefovir in DMSO formulation died without developing skin lesions (Fig. 5A). Treatment with cidofovir either in the DMSO or the gel formulation completely protected mice against virus-induced morbidity and mortality.

When tenofovir was evaluated in the DMSO formulation against HSV-2, there was again a statistically significant difference in the survival curves and morbidity curves between the group that received tenofovir starting the day of infection versus the placebo group. Treatment of HSV-2-infected mice with 1% adefovir or 1% cidofovir resulted in 80% and 100% protection against viral-induced morbidity and mortality, respectively (Fig. 5B). Similarly, when formulated in the gel at 1%, tenofovir delayed the appearance of herpes virus-related lesions and subsequent death of the animals (data not shown). Cidofovir formulated in gel resulted in 80% protection against HSV-2-induced morbidity and mortality while adefovir, under the same experimental conditions, afforded a significant delay in the appearance of lesions and subsequent death.

### Tenofovir is efficiently converted to its antivirally active metabolite in lymphocytes, epithelium and fibroblast cell cultures

The metabolic conversion of tenofovir to its antivirally active (diphosphorylated) metabolite was studied in human lymphocyte CEM (used for HIV infection experiments) and human epithelial TZM-bl and fibroblast HEL (used for HSV infection experiments) cell cultures according to previously established procedures (19). Treatment of cells with 2 µM [2,8-³H]tenofovir for 24 hrs at 72 hrs post initiation of the cultures resulted in the formation of tenofovir-diphosphate, reaching the levels of 13, 6 and 15 pmoles/10⁹ cells in T-lymphoid cells, epithelial cells, and fibroblasts, respectively. Thus, tenofovir is efficiently converted to its antivirally active metabolite in multiple different cell types that represent relevant target cells for either HIV or HSV infection in vivo.

### The active metabolite of tenofovir efficiently inhibits both HSV DNA polymerase and HIV reverse transcriptase

To become active as an inhibitor of virus-encoded herpes virus DNA polymerase or HIV-1 reverse transcriptase, tenofovir needs to be converted by cellular enzymes to its diphosphorylated derivative (tenofovir-DP). The active metabolite has been evaluated for its inhibitory activity against the target enzymes, using activated calf thymus DNA as the primer/template and [2,8-³H]dATP as the competing substrate. Tenofovir-DP efficiently inhibited both HIV-1 RT (IC₅₀: 4.3 µM) and HSV-1 DNA polymerase (IC₅₀: 1.3 µM). Thus, the antiherpetic activity of tenofovir in cell culture, organotypic epithelial raft cultures, human lymphoid and cervical ex vivo tissue, and virus-infected mice can be explained by the inhibition of the viral DNA polymerase by its active metabolite tenofovir-DP.

### Formulation (Vaginal gel)

| | (%w/w) |
|---|---|
| Tenofovir | 1.00 |
| Hydroxyethylcellulose, NF (Natrasol®250H) | 2.50 |
| Propylparaben, NF | 0.02 |
| Methylparaben, NF | 0.18 |
| Edetate Disodium, USP | 0.05 |
| Glycerin, USP | 20.00 |
| Citric Acid, USP | 1.00 |
| Purified Water, USP | 75.25 |
| Total | 100.00 |

Sodium hydroxide and hydrochloric acid are used as 10% w/w solutions to adjust pH to a target of 4.4. The methylparaben and propylparaben are dissolved in heated glycerin. Hydroxyethylcellulose is added and dispersed to form an organic phase. Edetate disodium and citric acid are dissolved in purified water, tenofovir is added and dispersed, pH adjusted to 4.4, and solution clarified by passage through a 0.22µm filter. Aqueous and organic phases are mixed, stirred well then filled into tubes or applicators.

### Safety and Tolerability

Tenofovir vaginal gel used 1% BID was well-tolerated in abstinent and sexually active HIV(-) and HIV(+) women, with limited systemic absorption and with possible beneficial effects on vaginal microflora.

### STUDY PROCEDURE

The object of the study was to evaluate the effectiveness of tenofovir gel when applied vaginally in preventing transmission of Herpes Simplex virus, in particular HSV-2.

The study was a Phase IIb trial - two-arm, double-blind, randomized, controlled trial, that compared the effect of 1% tenofovir gel with a placebo gel among 889 sexually active women aged from 18-40 years old, at high risk for sexually transmitted HIV infection.

The placebo gel (known as the 'universal' placebo gel) was formulated to minimize any possible effects - negative or positive - on study endpoints. It is isotonic to avoid epithelial cell swelling or dehydration. It was formulated at a pH of 4-5 but has minimal buffering capacity. When mixed with an equal volume of semen, the placebo gel induced only a trivial decrease in semen pH (from 7.8 to 7.7). The placebo gel contained hydroxyethylcellulose (HEC) as a gelling agent, and its viscosity is comparable to that of tenofovir gel. The gel does not have anti-HIV or anti-HSV-2 properties. The gel contained sorbic acid as a preservative. Sorbic acid has no anti-HIV activity and is readily metabolized by human cells. The placebo gel was formulated as follows:

| Chemical Name | %w/w | Quantity |
|---|---|---|
| Purified water | 96.34 | 674.4 Liters |
| Sodium Chloride | 0.85 | 6.545 Kg |
| Hydroxycellulose NF | 2.7 | 20.79 Kg |
| Sorbic acid EP/NF | 0.10 | 770.0 g |
| Sodium hydroxide NF (qs to pH 4.4) | 0.0020 | 15.4 g |
| Sodium hydroxide NF | 0.0080 | 61.6 g |

The participants were trained in proper methods of storing and applying the assigned study product. Participants were instructed to insert one dose (the entire contents of one applicator) of product into the vagina up to 12 hours before each act of vaginal intercourse (intercourse may take place immediately after product insertion) and insert a second dose as soon as possible after coitus but within 12 hours.

The study participants were advised to:
- Only apply the assigned product vaginally.
- Not douche or otherwise clean the vagina, or insert other objects or vaginal products, for 2 hours after gel insertion. If a woman planned to douche after coitus, she was advised to insert the gel after douching.
- Properly store their study products (in a cool dry place out of direct sunlight)
- To use study product whether or not a condom is used.

The study participants completed monthly follow-up visits for the duration of their participation, and blood was drawn at enrolment, then at pre-specified points during follow up and at study exit, in order to test for HIV and HSV-2.

### Results

The presence of HSV-2 was determined in the blood samples drawn using the Kalon HSV-2 type specific EIA (Kolon Biological Ltd., United Kingdom). At enrolment 454 of 888 women (1 woman had no archived blood for testing) had pre-existing HSV-2 infection resulting in a prevalence of 51.1 %. The remaining 434 women were regarded as HSV-2 susceptible at entry into the trial, and exit HSV-2 status was determined in 426 of these women. It should be noted that 4 of these women had no archived study exit specimen and 4 had indeterminate HSV-2 results at study exit.

Of the 205 women using the tenofovir formulation for the duration of the study, 29 became infected with HSV-2. Of the 224 women provided with the placebo for the duration of the study, 58 became infected with HSV-2. Thus the tenofovir gel provided 51% protection against HSV-2

| | Tenofovir Formulation | Placebo |
|---|---|---|
| | No. of Participants = 202 | No. of Participants = 224 |
| No. of HSV-2 infections | 29 | 58 |
| Women-Years of follow up | 293.3 | 287.3 |
| Rate of HSV-2 infection per 100 women | 9.9 | 20.2 |

Although certain embodiments have been described in detail above, those having ordinary skill in the art will clearly understand that many modifications are possible in the embodiments without departing from the scope of the claims.

## Claims

1. The antiviral compound (R)-(1-(6-amino-9H-purin-9-yl)propan-2-yloxy)methylphosphonic acid (tenofovir) for use in the reduction of or prevention of the transmission of HSV-2 to a mammal wherein tenofovir is applied vaginally to a human female as a gel formulation, which comprises
| Ingredient | (%w/w) |
|---|---|
| Tenofovir | 0.2-2.00 |
| Hydroxyethylcellulose, NF (Natrasol®250H) | 1-5.0 |
| Propylparaben, NF | 0.01-0.10 |
| Methylparaben, NF | 0.1-0.25 |
| Edetate Disodium, USP | 0.02-0.10 |
| Glycerin, USP | 3.00-30.00 |
| Citric Acid, USP | 0.5-2.00 |
| Purified Water, USP | to 100%. |

2. The antiviral compound (R)-(1-(6-amino-9H-purin-9-yl)propan-2-yloxy)methylphosphonic acid (tenofovir) for use in the reduction of or prevention of the acquisition of HSV-2 by a mammal, wherein tenofovir is applied vaginally to a human female as a gel formulation, which comprises
| Ingredient | (%w/w) |
|---|---|
| Tenofovir | 0.2-2.00 |
| Hydroxyethylcellulose, NF (Natrasol®250H) | 1-5.0 |
| Propylparaben, NF | 0.01-0.10 |
| Methylparaben, NF | 0.1-0.25 |
| Edetate Disodium, USP | 0.02-0.10 |
| Glycerin, USP | 3.00-30.00 |
| Citric Acid, USP | 0.5-2.00 |
| Purified Water, USP | to 100%. |

3. The antiviral compound for use according to claim 1 or 2, wherein the formulation is applied before sexual activity.

4. The antiviral compound for use according to claim 1 or 2, wherein the formulation is applied after sexual activity.

5. The antiviral compound for use according to claim 1 or 2, wherein the formulation is applied both before and after sexual activity.

6. The antiviral compound for use according to claim 1 or 2, wherein the formulation is applied once or twice daily.

7. The antiviral compound for use according to claim 1 or 2, wherein sodium hydroxide and hydrochloric acid are added to adjust the pH to 4.4.

8. The antiviral compound for use according to claim 1or 2, wherein the formulation comprises:
| | (%w/w) |
|---|---|
| Tenofovir | 1.00 |
| Hydroxyethylcellulose, NF (Natrasol®□250H) | 2.50 |
| Propylparaben, NF | 0.02 |
| Methylparaben, NF | 0.18 |
| Edetate Disodium, USP | 0.05 |
| Glycerin, USP | 20.00 |
| Citric Acid, USP | 1.00 |
| Purified Water, USP | 75.25 |
| Total | 100.00 |

9. The antiviral compound for use according to claim 8, wherein sodium hydroxide and hydrochloric acid are added to adjust the pH to 4.4.

10. The antiviral compound for use according to claim 9, wherein the formulation is coated upon a condom.

## Patentansprüche

1. Antivirale Verbindung (R)-(1-(6-Amino-9H-purin-9-yl)propan-2-yloxy)methylphosphonsäure (Tenofovir) für die Verwendung bei dem Verringern oder Verhindern der Übertragung von HSV-2 an einen Säuger, wobei Tenofovir bei einer menschlichen Frau vaginal als Gel-Formulierung angewendet wird, die umfasst:
| Inhaltsstoff | (% w/w) |
|---|---|
| Tenofovir | 0,2-2,00 |
| Hydroxyethylcellulose, NF (Natrasol®250H) | 1-5,0 |
| Propylparaben, NF | 0,01-0,10 |
| Methylparaben, NF | 0,1-0,25 |
| Dinatriumedetat, USP | 0,02-0,10 |
| Glycerin, USP | 3,00-30,00 |
| Citronensäure, USP | 0,5-2,00 |
| Gereinigtes Wasser, USP | auf 100 %. |

2. Antivirale Verbindung (R)-(1-(6-Amino-9H-purin-9-yl)propan-2-yloxy)methylphosphonsäure (Tenofovir) für die Verwendung bei dem Verringern oder Verhindern des Erwerbens von HSV-2 durch einen Säuger, wobei Tenofovir bei einer menschlichen Frau vaginal als Gel-Formulierung angewendet wird, die umfasst:
| Inhaltsstoff | (% w/w) |
|---|---|
| Tenofovir | 0,2-2,00 |
| Hydroxyethylcellulose, NF (Natrasol®250H) | 1-5,0 |
| Propylparaben, NF | 0,01-0,10 |
| Methylparaben, NF | 0,1-0,25 |
| Dinatriumedetat, USP | 0,02-0,10 |
| Glycerin, USP | 3,00-30,00 |
| Citronensäure, USP | 0,5-2,00 |
| Gereinigtes Wasser, USP | auf 100 %. |

3. Antivirale Verbindung für die Verwendung gemäß Anspruch 1 oder 2, wobei die Formulierung vor sexueller Aktivität angewendet wird.

4. Antivirale Verbindung für die Verwendung gemäß Anspruch 1 oder 2, wobei die Formulierung nach sexueller Aktivität angewendet wird.

5. Antivirale Verbindung für die Verwendung gemäß Anspruch 1 oder 2, wobei die Formulierung sowohl vor als auch nach sexueller Aktivität angewendet wird.

6. Antivirale Verbindung für die Verwendung gemäß Anspruch 1 oder 2, wobei die Formulierung einmal oder zweimal täglich angewendet wird.

7. Antivirale Verbindung für die Verwendung gemäß Anspruch 1 oder 2, wobei Natriumhydroxid und Salzsäure zugegeben sind, um den pH-Wert auf 4,4 einzustellen.

8. Antivirale Verbindung für die Verwendung gemäß Anspruch 1 oder 2, wobei die Formulierung umfasst:
| | (% w/w) |
|---|---|
| Tenofovir | 1,00 |
| Hydroxyethylcellulose, NF (Natrasol®250H) | 2,50 |
| Propylparaben, NF | 0, 02 |
| Methylparaben, NF | 0,18 |
| Dinatriumedetat, USP | 0,05 |
| Glycerin, USP | 20,00 |
| Citronensäure, USP | 1,00 |
| Gereinigtes Wasser, USP | 75,25 |
| Gesamt | 100,00 |

9. Antivirale Verbindung für die Verwendung gemäß Anspruch 8, wobei Natriumhydroxid und Salzsäure zugegeben sind, um den pH-Wert auf 4,4 einzustellen.

10. Antivirale Verbindung für die Verwendung gemäß Anspruch 9, wobei die Formulierung auf ein Kondom aufgeschichtet ist.

## Revendications

1. Composé antiviral acide (R)-(1-(6-amino-9H-purin-9-yl)propan-2-yloxy)méthylphosphonique (ténofovir) pour utilisation dans la réduction ou la prévention de la transmission de HSV-2 à un mammifère, le ténofovir étant appliqué par voie vaginale à une femelle humaine sous la forme d'une formulation de gel, qui comprend
| Composant | (% m/m) |
|---|---|
| Ténofovir Hydroxyéthylcellulose, NF | 0,2-2,00 |
| (Natrasol®250H) | 1-5,0 |
| Propylparabène, NF | 0,01-0,10 |
| Méthylparabène, NF | 0,1-0,25 |
| Édétate disodique, USP | 0,02-0,10 |
| Glycérine, USP | 3,00-30,00 |
| Acide citrique, USP | 0,5-2,00 |
| Eau purifiée, USP | q.s. 100 %. |

2. Composé antiviral acide (R)-(1-(6-amino-9H-purin-9-yl)propan-2-yloxy)méthylphosphonique (ténofovir) pour utilisation dans la réduction ou la prévention de l'acquisition de HSV-2 par un mammifère, le ténofovir étant appliqué par voie vaginale à une femelle humaine sous la forme d'une formulation de gel, qui comprend
| | |
|---|---|
| Composant | (% m/m) |
| Ténofovir | 0,2-2,00 |
| Hydroxyéthylcellulose, NF (Natrasol®250H) | 1-5,0 |
| Propylparabène, NF | 0,01-0,10 |
| Méthylparabène, NF | 0,1-0,25 |
| Édétate disodique, USP | 0,02-0,10 |
| Glycérine, USP | 3,00-30,00 |
| Acide citrique, USP | 0,5-2,00 |
| Eau purifiée, USP | q.s. 100 %. |

3. Composé antiviral pour utilisation selon la revendication 1 ou 2, **caractérisé en ce que** la formulation est appliquée avant une activité sexuelle.

4. Composé antiviral pour utilisation selon la revendication 1 ou 2, **caractérisé en ce que** la formulation est appliquée après une activité sexuelle.

5. Composé antiviral pour utilisation selon la revendication 1 ou 2, **caractérisé en ce que** la formulation est appliquée à la fois avant et après une activité sexuelle.

6. Composé antiviral pour utilisation selon la revendication 1 ou 2, **caractérisé en ce que** la formulation est appliquée une ou deux fois par jour.

7. Composé antiviral pour utilisation selon la revendication 1 ou 2, **caractérisé en ce que** de l'hydroxyde de sodium et de l'acide chlorhydrique sont ajoutés pour ajuster le pH à 4,4.

8. Composé antiviral pour utilisation selon la revendication 1 ou 2, **caractérisé en ce que** la formulation comprend :
| | (% m/m) |
|---|---|
| Ténofovir | 1,00 |
| Hydroxyéthylcellulose, NF | 2,50 |
| (Natrasol®250H) | |
| Propylparabène, NF | 0, 02 |
| Méthylparabène, NF | 0,18 |
| Édétate disodique, USP | 0,05 |
| Glycérine, USP | 20,00 |
| Acide citrique, USP | 1,00 |
| Eau purifiée, USP | 75,25 |
| Total | 100,00 |

9. Composé antiviral pour utilisation selon la revendication 8, **caractérisé en ce que** de l'hydroxyde de sodium et de l'acide chlorhydrique sont ajoutés pour ajuster le pH à 4,4.

10. Composé antiviral pour utilisation selon la revendication 9, **caractérisé en ce que** la formulation est revêtue sur un préservatif.
